# EUROPEAN PATENT APPLICATION

(11) **EP 4 475 278 A1**
(43) Date of publication of application: **11.12.2024**
(21) Application number: 24172376.6
(22) Date of filing: 25.04.2024
(51) Int. Cl.: H01M 10/615, H01M 10/48, H01M 10/42, H01M 10/657, H01M 10/0562, H01M 10/052, B60K 1/04, B60L 50/64, G01N 31/22, H01M 10/44, H01M 10/625, H01M 10/63, H01M 50/569, H01M 50/572

(54) **BATTERY SYSTEM**

(30) Priority: 09.06.2023 JP 2023095604
(71) Applicant: TOYOTA JIDOSHA KABUSHIKI KAISHA, Toyota-shi, Aichi-ken, 471-8571 (JP)
(72) Inventor: HAGIWARA, Hideki, Toyota-shi, 471-8571 (JP); NAGASE, Hiroshi, Toyota-shi, 471-8571 (JP); OGUMA, Yasumasa, Toyota-shi, 471-8571 (JP); YOSHIDA, Jun, Toyota-shi, 471-8571 (JP); MIGITA, Tsubasa, Toyota-shi, 471-8571 (JP); UCHIDA, Yoshihiro, Toyota-shi, 471-8571 (JP)
(74) Representative: D Young & Co LLP

(57) **Abstract**

A battery system includes a battery unit, a heater unit, and a sensor unit. The battery unit is accommodated in a battery case and includes sulfide-based all-solid-state batteries. The heater unit is configured to heat the battery unit. The sensor unit is configured to measure a hydrogen sulfide gas concentration inside the battery case. The heater unit is started to be driven when a measured value of the sensor unit becomes greater than the first threshold value. After starting the driving of the heater unit, when the measured value becomes greater than the second threshold value that is higher than the first threshold value, a responsive control is executed to deal with generation of the hydrogen sulfide gas from the sulfide-based all-solid-state batteries.

## Description

### BACKGROUND OF THE INVENTION

### 1. Field of the Invention

The present invention relates to a battery system.

### 2. Description of Related Art

Japanese Unexamined Application Publication No. 2022-46077 discloses a battery system including secondary batteries, a sensor, and a controller. The secondary batteries can be sulfide-based all-solid-state batteries and are accommodated in a battery case. The sensor is, for example, a hydrogen sulfide densitometer and detects (measures) a hydrogen sulfide gas concentration inside the battery case. When a detected value of the sensor is higher than a threshold value, the controller determines that hydrogen sulfide is generated from the secondary batteries.

### SUMMARY OF THE INVENTION

Sulfide-based all-solid-state batteries can generate hydrogen sulfide gas inside the battery case. Inside the battery case, hydrogen sulfide gas can be present, and miscellaneous gases different from the hydrogen sulfide gas can also be present. When the sensor reacts to the miscellaneous gases, measurement errors in the sensor can be increased. As a result, the measured value of the sensor might become greater than the threshold value even when hydrogen sulfide is not actually generated from the sulfide-based all-solid-state batteries. In this case, it might be erroneously determined that the hydrogen sulfide gas is generated from the sulfide-based all-solid-state batteries. Consequently, control, which is originally unnecessary, might be executed, so that the hydrogen sulfide gas from the sulfide-based all-solid-state batteries might be dealt with excessively.

The present disclosure has been made in order to solve the above problems, and an object of the present disclosure is to avoid that hydrogen sulfide gas from sulfide-based all-solid-state batteries is dealt with excessively.

A battery system of the present disclosure includes a battery unit, a heater unit, and a sensor unit. The battery unit is accommodated in a battery case and includes a sulfide-based all-solid-state battery. The heater unit is configured to heat the battery unit. The sensor unit is configured to measure a hydrogen sulfide gas concentration inside the battery case. The heater unit is started to be driven when a measured value of the sensor unit becomes greater than the first threshold value. After starting the driving of the heater unit, when the measured value becomes greater than the second threshold value that is higher than the first threshold value, a responsive control is executed to deal with generation of the hydrogen sulfide gas from the sulfide-based all-solid-state battery.

According to the present disclosure, it is possible to avoid that hydrogen sulfide gas from the sulfide-based all-solid-state batteries is dealt with excessively.

### BRIEF DESCRIPTION OF THE DRAWINGS

Features, advantages, and technical and industrial significance of exemplary embodiments of the invention will be described below with reference to the accompanying drawings, in which like signs denote like elements, and wherein:
FIG. 1 is a view schematically showing the overall configuration of a vehicle including a battery system according to an embodiment of the present disclosure;
FIG. 2 is a view schematically showing each cell of an assembled battery of a battery unit;
FIG. 3 is a view explaining the connection relationship among the battery unit, an SMR (system main relay), and a drive unit;
FIG. 4 is a perspective view of the battery case and the battery unit;
FIG. 5 shows a section taken along line A-A in FIG. 4;
FIG. 6 is a flowchart exemplifying processing and control executed by an ECU (electronic control unit) in the embodiment; and
FIG. 7 is a flowchart exemplifying processing and control executed by the ECU in a modification.

### DETAILED DESCRIPTION OF EMBODIMENTS

Hereinafter, embodiments of the present disclosure will be described in detail with reference to the drawings. The same or corresponding parts in the drawings are designated by the same reference numerals, and the description thereof will not be repeated. The embodiments and their modifications may be combined with each other as appropriate.

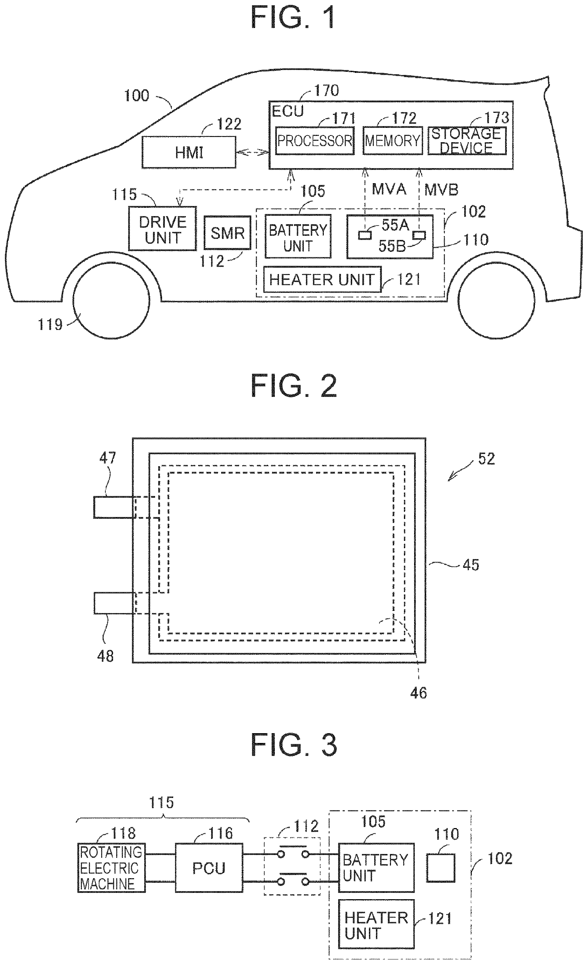

FIG. 1 is a view schematically showing the overall configuration of a vehicle including a battery system according to an embodiment of the present disclosure. This vehicle is a battery electric vehicle (BEV), for example, and may also be another type of an electrified vehicle, such as a hybrid electric vehicle (HEV) including an engine.

With reference to FIG. 1, a vehicle 1 includes a battery case 102, a battery unit 105, a sensor unit 110, an SMR 112, a drive unit 115, driving wheels 119, a heater unit 121, a human machine interface (HMI) device 122, and an ECU 170. The battery case 102, the battery unit 105, the sensor unit 110, the SMR 112, the heater unit 121, the HMI device 122, and the ECU 170 form a "battery system" of the present disclosure.

The battery case 102 accommodates the battery unit 105. The battery unit 105 is configured to be chargeable and dischargeable, and stores electric power for use in traveling of the vehicle 1. The battery unit 105 includes sulfide-based all-solid-state batteries. The all-solid-state battery refers to a battery having a solid electrolyte layer. The sulfide-based all-solid-state battery is an all-solid-state battery in which at least one of the material of a positive electrode active material layer of the battery unit 105 and the material of a solid electrolyte layer of the battery unit 105 contains a sulfur component. The battery unit 105 includes a plurality of (in this example, two) assembled batteries.

FIG. 2 is a view schematically showing each cell of the assembled battery of the battery unit 105. With reference to FIG. 2, each cell 52 includes a laminated film 45, an electrode body 46, a positive electrode tab 47, and a negative electrode tab 48. The electrode body 46 is sealed inside the laminated film 45. The positive electrode tab 47 and the negative electrode tab 48 are connected to the electrode body 46 and are drawn out from the inside of the laminated film 45. Each cell 52 is a sulfide-based all-solid-state battery.

When charging-discharging of the battery unit 105 is repeated, hydrogen sulfide gas can be generated inside the battery cells 25. For example, gaps are formed in portions of the positive electrode tab 47 and the negative electrode tab 48, which are pulled out from the laminated film 45, and air might infiltrate into the laminated film 45 through the gaps. As a result, the solid electrolyte layer and water in the air might react with each other, thereby generating hydrogen sulfide gas. In another case, an internal short circuit might be caused inside the electrode body 46, and the electrode body 46 can have a high temperature, thereby generating hydrogen sulfide gas. In this manner, the sulfide-based all-solid-state battery can generate hydrogen sulfide gas from itself. Such generation of hydrogen sulfide gas is unique to a sulfide-based all-solid-state battery, and is accelerated as the cell temperature becomes higher (the higher the cell temperature, the more the hydrogen sulfide gas is generated).

FIG. 3 is a view explaining the connection relationship among the battery unit 105, the SMR 112, and the drive unit 115. With reference to FIG. 3, the battery unit 105 is configured to be connectable to the drive unit 115 via the SMR 112. Organic solvents can be present outside the battery unit 105. The battery case 102 accommodates, in addition to the battery unit 105, components formed of various chemical materials including silicone rubber materials.

The SMR 112 is connected between the battery unit 105 and the drive unit 115. Closing the SMR 112 corresponds to turning on the driving system of the vehicle 1. Opening the SMR 112 corresponds to turning off the driving system of the vehicle 1.

With reference to FIG. 1 again, the sensor unit 110 includes sensors 55A, 55B. Each of the sensors 55A, 55B is configured to measure a concentration of hydrogen sulfide (H2S) gas inside the battery case 102. Measured values of the sensors 55A, 55B are also expressed as measured values MVA and MVB, respectively. Each sensor can also react to different gases (miscellaneous gases) from the hydrogen sulfide gas. That is, each of the measured values MVA and MVB can also be affected by the miscellaneous gases.

The drive unit 115 includes a power control unit (PCU) 116 and a rotating electric machine 118 (see FIG. 3). The PCU 116 is connected to the rotating electric machine 118. The PCU 116 is an electric power converter that converts direct current electric power supplied from the battery unit 105 via the SMR 112 into alternating current electric power, and supplies the alternating current electric power to the rotating electric machine 118. The rotating electric machine 118 is connected to the driving wheel 119 and receives the alternating current electric power from the PCU 116 to generate propulsive force (traveling driving force) for the vehicle 1. The heater unit 121 includes a plurality of (in this example, two) heaters and is configured to heat the battery unit 105.

The HMI device 122 is a touch screen device, and functions as a notification device that receives various operations from a user of the vehicle 1 and provides notification to the user by displaying various screens thereon.

The ECU 170 includes a processor 171, a memory 172, and a storage device 173. The processor 171 is, for example, a central processing unit (CPU), and executes various calculation processing. The memory 172 includes a read only memory (ROM) and a random access memory (RAM) (both not shown). The ROM stores programs executed by the processor 171. The storage device 173 stores various data.

The ECU 170 controls various devices of the vehicle 1, such as the SMR 112, the PCU 116, the heater unit 121, the HMI device 122, and a solenoid on-off valve (described later). The ECU 170 opens and closes the SMR 112. The ECU 170 controls the PCU 116 and thereby to control charging electric power and discharging electric power of the battery unit 105. The ECU 150 sets an upper limit of the charging electric power and an upper limit of the discharging electric power. The ECU 150 sets these upper limits to be smaller and thereby to restrict the charging electric power and the discharging electric power. When the discharging electric power and the discharging electric power (charging-discharging of the battery unit 105) are restricted, generation of the hydrogen sulfide gas is reduced.

The ECU 170 determines whether or not the hydrogen sulfide gas from the battery unit 105 is generated inside the battery case 102 based on the measured values MVA and MVB. For example, when both measured values MVA and MVB are less than a predetermined threshold value (described later), the ECU 170 determines that the hydrogen sulfide gas is not generated inside the battery case 102. On the other hand, when at least one of the measured values MVA and MVB is equal to or greater than the above threshold value, the ECU 170 determines that the hydrogen sulfide gas is generated inside the battery case 102. In this case, the ECU 170 executes a responsive control (described in detail later) to deal with the generation of the hydrogen sulfide gas from the battery unit 105.

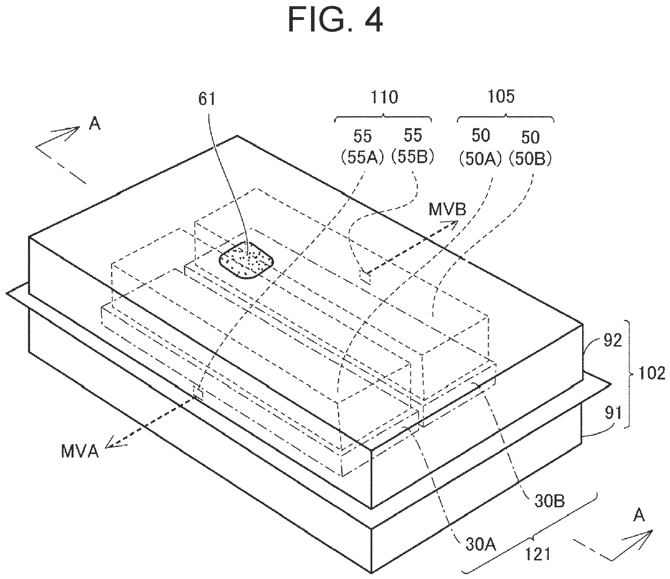

FIG. 4 is a perspective view of the battery case 102 and the battery unit 105. With reference to FIG. 4, the battery case 102 accommodates two assembled batteries 50 (batteries 50A, 50B) included in the battery unit 105. The battery case 102 includes a lower case 91 and an upper case 92. The upper case 92 is formed with a respiratory membrane 61.

The heater unit 121 includes heaters 30A, 30B. The heaters 30A, 30B are arranged below the batteries 50A, 50B, respectively. The heaters 30A, 30B heat the batteries 50A, 50B, respectively.

The sensors 55A, 55B are arranged in the vicinities of the batteries 50A, 50B, respectively. In other words, the sensor 55A is closer to the battery 50A than the sensor 55B is, and the sensor 55B is closer to the battery 50B than the sensor 55A is.

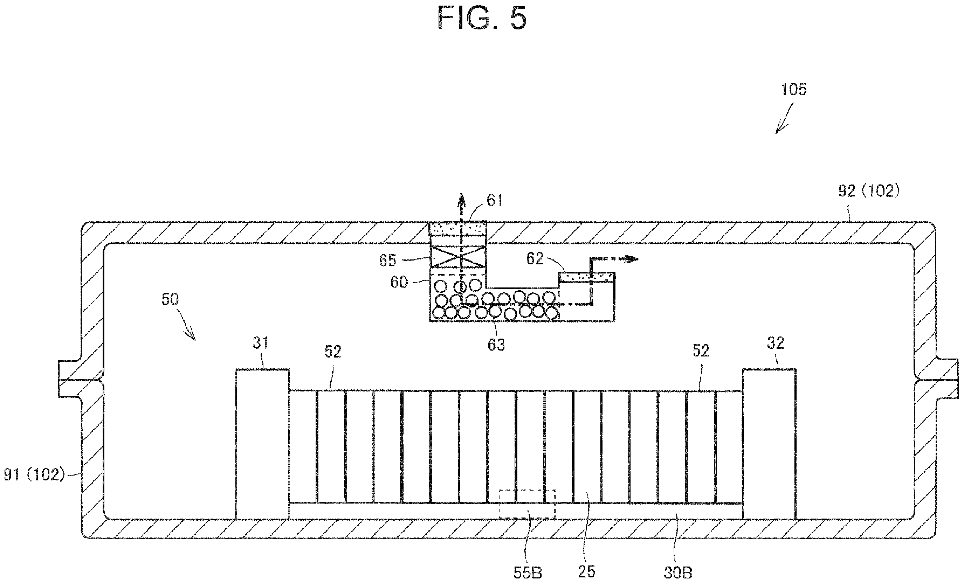

FIG. 5 shows a section taken along line A-A in FIG. 4. With reference to FIG. 5, the assembled battery 50 (in this example, the battery 50B) includes a plurality of cells 52. The plurality of cells 52 and the heater 30B are arranged between end plates 31, 32. The sensor 55B is disposed on the bottom surface of the lower case 91. This is because the hydrogen sulfide gas is heavier than the air.

The battery case 102 (upper case 92) is provided with a duct 60, which is a communication passage that communicates the inside and the outside of the battery case 102. The duct 60 is provided with respiratory membranes 61, 62. Each of the respiratory membranes 61, 62 is made of an air-permeable and waterproof material. This material is, for example, a GORE-TEX (registered trademark) material. The internal space of the battery case 102 is almost sealed, but is not completely sealed because the duct 60 is provided with the respiratory membranes 61, 62. A desulfurizing agent 63 is disposed inside the duct 60.

When the pressure inside the battery case 102 increases, the air is exhausted from the inside to the outside of the battery case 102 through the duct 60 and the respiratory membranes 61, 62. When the hydrogen sulfide gas is present inside the battery case 102, the desulfurizing agent 63 adsorbs and purifies the hydrogen sulfide gas flowing from the inside to the outside of the battery case 102. On the other hand, when the pressure inside the battery case 102 decreases, the air flows from the outside to the inside of the battery case 102 through the duct 60 and the respiratory membranes 61, 62.

The duct 60 is provided with a solenoid on-off valve 65. The opening degree of the solenoid on-off valve 65 is adjusted in accordance with a command from the ECU 170. By adjusting this opening degree, the duct 60 can be opened or closed. Each of the duct 60 and the solenoid on-off valve 65 is included in the aforementioned battery system.

As described above, the battery unit 105 (sulfide-based all-solid-state batteries) can generate the hydrogen sulfide gas inside the battery case 102. Inside the battery case 102, the hydrogen sulfide gas can be present, and the miscellaneous gases different from the hydrogen sulfide gas can also be present. This is because the air flowing from the outside to the inside of the battery case 102 through the duct 60 contains the miscellaneous gases, or the miscellaneous gases are generated from various materials accommodated in the battery case 102, organic solvents outside of the battery unit 105 (assembled batteries 50), or the materials configuring the respiratory membranes 61, 62.

Each of the sensors 55A, 55B can react to the miscellaneous gases. In this case, measurement errors included in the measured values MVA and MVB might increase. Consequently, even though the hydrogen sulfide gas is not actually generated from the battery unit 105, at least one of the measured values MVA and MVB might be greater than the above-described threshold value. Hence, the hydrogen sulfide gas might be erroneously determined to be generated from the battery unit 105. Consequently, control, which is originally unnecessary, might be executed, so that the hydrogen sulfide gas might be dealt with excessively. In order to avoid the above erroneous determination, it seems to be preferable to provide the vehicle 1 with a high-precision hydrogen sulfide gas concentration sensor, which has low reactivity to the miscellaneous gases (only the hydrogen sulfide gas can be measured with high precision). However, this solution causes increase in cost.

In the embodiment, the ECU 170 has a configuration to deal with the above problems. Specifically, the ECU 170 starts to drive the heater unit 121 (e.g., both heaters 30A, 30B) when at least one of the measured values MVA and MVB becomes greater than the first threshold value. After starting the driving of the heater unit 121, neither the measured values MVA nor MVB becomes greater than the second threshold value, the ECU 170 determines that the hydrogen sulfide gas is not generated from the battery unit 105 inside the battery case 102. On the other hand, when at least one of the measured values MVA and MVB becomes greater than the second threshold value, the ECU 170 determines that the hydrogen sulfide gas is generated from the battery unit 105 inside the battery case 102. Based on the result of this determination, the ECU 170 executes the responsive control (described in detail later) to deal with the generation of the hydrogen sulfide gas from the battery unit 105. The second threshold value is higher than the first threshold value.

By configuring as described above, when the battery unit 105 actually generates the hydrogen sulfide gas, the generation of the hydrogen sulfide gas is accelerated due to heating of the battery unit 105 (increase in cell temperature) after starting the driving of the heater unit 121. The amount of the hydrogen sulfide gas generated from the battery unit 105 increases, and the hydrogen sulfide gas concentration inside the battery case 102 further increases. As a result, at least one of the measured values MVA and MVB becomes greater than the second threshold value. In the meantime, the concentration of the miscellaneous gases inside the battery case 102 has no relation with the increase in temperature of the battery unit 105; therefore, the concentration does not increase in response to starting the driving of the heater unit 121. According to the above configuration, it is possible to avoid an erroneous determination that the hydrogen sulfide gas is generated. Furthermore, it is not necessarily required to use a high-precision hydrogen sulfide gas concentration sensor. In addition, it is possible to avoid that the responsive control is unnecessarily started due to the reaction of the sensors 55A, 55B to the miscellaneous gases. Accordingly, it is possible to avoid such a situation that the hydrogen sulfide gas generated from the battery unit 105 is dealt with excessively.

An example of the responsive control will be described below. The responsive control includes at least one of; opening the SMR 112 (turning off the driving system); opening the solenoid on-off valve 65; controlling the HMI device 122 to notify the user of generation of the hydrogen sulfide gas inside the battery case 102; and controlling the PCU 116 to restrict the charging-discharge of the battery unit 105.

By opening the SMR 112, the battery unit 105 can be separated from the PCU 116. Thereby, the battery unit 105 becomes electrically independent and its conduction with the other components of the vehicle 1 is cut off. As a result, the charging-discharging is stopped (the vehicle 1 is stopped), and the generation of the hydrogen sulfide gas is reduced. By opening the solenoid on-off valve 65, the hydrogen sulfide gas can be discharged from the inside to the outside of the battery case 102 through the duct 60. By controlling the HMI device 122 as described above, the user is notified of the generation of the hydrogen sulfide gas. Accordingly, the user can be motivated to deal with the generation of the hydrogen sulfide gas (for example, to use the maintenance service for the vehicle 1). By controlling the PCU 116 as described above, the charging-discharging of the battery unit 105 is restricted. Thereby, the generation of the hydrogen sulfide gas is reduced. In this manner, according to the responsive control, it is possible to deal with the generation of the hydrogen sulfide gas inside the battery case 102.

The first threshold value is equal to or less than 1 ppm (1 ppm = 0.0001%), for example. The second threshold value is greater than 1 ppm and less than 5 ppm, for example. In the embodiment, the first threshold value is set to be 1 ppm and the second threshold value is set to be 3 ppm.

Generally, the control concentration and the permissible concentration of the hydrogen sulfide gas should be 1 ppm and 5 ppm, respectively. With the above configuration, even after at least one of the measured values MVA and MVB becomes greater than the second threshold value (3 ppm) after starting the driving of the heater unit 121, it is possible to avoid that the hydrogen sulfide gas concentration becomes more than the permissible concentration (5 ppm). Thereby, it is possible to accurately determine whether or not the hydrogen sulfide gas is generated without causing the hydrogen sulfide gas concentration to become more than the permissible concentration.

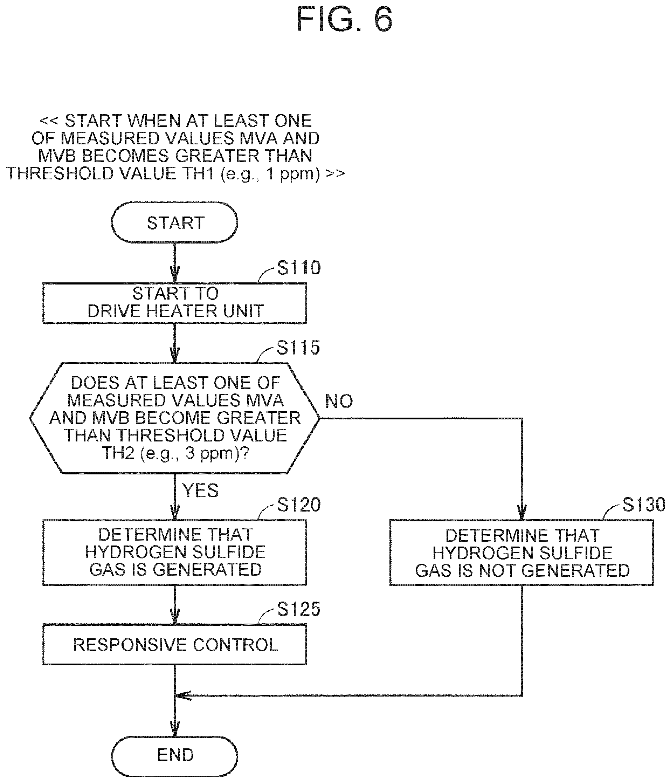

FIG. 6 is a flowchart exemplifying the processing and the control executed by the ECU 170 in the embodiment. This flowchart is started when at least one of the measured values MVA and MVB becomes greater than a threshold value TH1. The threshold value TH1 corresponds to the first threshold value.

With reference to FIG. 6, the ECU 170 starts to drive the heater unit 121 (S 110), and thereafter determines whether or not at least one of the measured values MVA and MVB becomes greater than a threshold value TH2 (S115). The threshold value TH2 corresponds to the second threshold value.

When at least one of the measured values MVA and MVB becomes greater than the threshold value TH2 (YES in S115), the ECU 170 determines that the hydrogen sulfide gas is generated inside the battery case 102 (S120), and executes the above-described responsive control (S125). On the other hand, when neither the measured values MVA nor MVB becomes greater than the threshold value TH2 (NO in S115), the ECU 170 determines that the hydrogen sulfide gas is not generated inside the battery case 102 (S130). In this case, it can be considered that the sensor unit 110 simply has simply reacted to the miscellaneous gases. After S125 or S130, the process is ended.

As described above, according to the embodiment, it is possible to determine whether or not the hydrogen sulfide gas is generated with high precision. In addition, it is possible to avoid that the hydrogen sulfide gas generated from the battery unit 105 is dealt with excessively.

### Modification of embodiment

With reference to FIG. 4 again, in the embodiment, the ECU 170 drives both heaters 30A, 30B; however, from the viewpoint of reduction in electric power consumption, only one of these heaters may be driven. In this modification, the ECU 170 starts to drive only the heater 30A of the heaters 30A, 30B when the measured value MVA becomes greater than the threshold value TH1 earlier than the measured value MVB does.

As mentioned above, the sensor 55A is disposed closer to the battery 50A than the sensor 55B is. Therefore, when the measured value MVA becomes greater than the threshold value TH1 earlier than the measured value MVB does, it can be considered that the hydrogen sulfide gas is generated from the battery 50A rather than the battery 50B. Therefore, in order to promote the generation of the hydrogen sulfide gas from the battery 50A (battery unit 105), it is sufficient to start to drive only the heater 30A of the heaters 30A, 30B; and it may also be considered that it is not necessarily required to drive both heaters. By starting to drive the heater 30A alone as described above, reduction in electric power consumption in the heater unit 121 can be attained compared to the embodiment in which both heaters 30A, 30B are driven. Furthermore, after starting the driving of the heater 30A, the generation of the hydrogen sulfide gas from the battery 50A is promoted, and the measured value MVA can become greater than the threshold value TH2. As a result, it is determined that the hydrogen sulfide gas is generated from the battery 50A, and the responsive control is then executed.

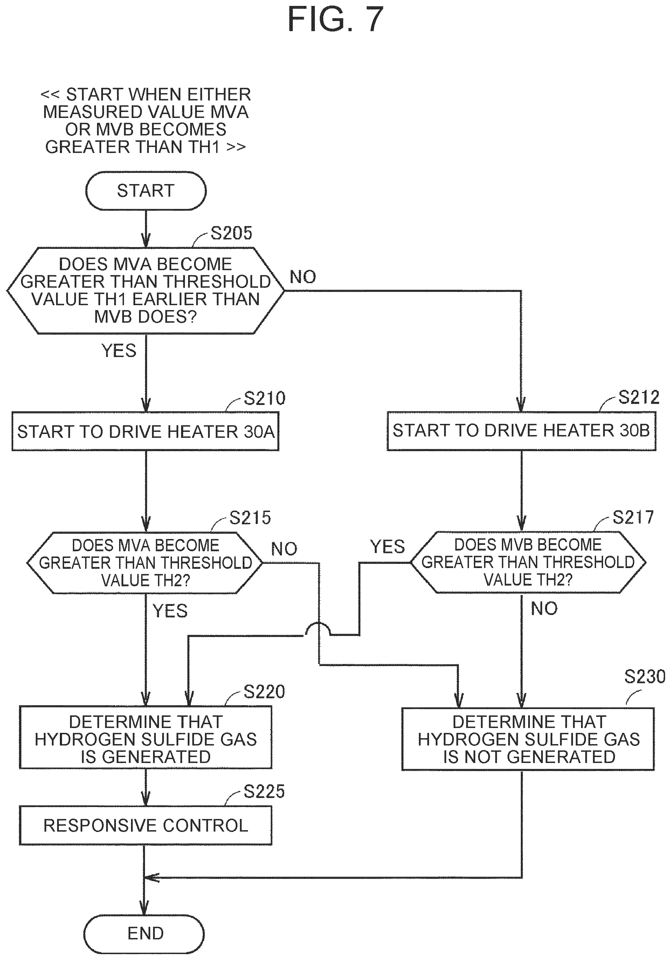

FIG. 7 is a flowchart exemplifying the processing and the control performed by the ECU 170 in this modification. This flowchart is started when either the measured values MVA or MVB becomes greater than the threshold value TH1.

With reference to FIG. 7, the ECU 170 determines whether or not the measured value MVA becomes greater than the threshold value TH1 earlier than the measured value MVB does; that is, whether or not the measured value greater than the threshold value TH1 is the measured value MVA (S205).

When the measured value MVA becomes greater than the threshold value TH1 earlier than the measured value MVB does (YES in S205), the ECU 170 starts to drive the heater 30A (S210), and then determines whether or not the measured value MVA becomes greater than the threshold value TH2 (S215). When the measured value MVA does not become greater than the threshold value TH2 (NO in S215), the ECU 170 determines that the hydrogen sulfide gas is not generated inside the battery case 102 (S230). When the measured value MVA becomes greater than the threshold value TH2 (YES in S215), the ECU 170 determines that the hydrogen sulfide gas is generated (S220), and then executes the responsive control (S225).

When the measured value MVB becomes greater than the threshold value TH1 earlier than the measured value MVA does; that is, when the measured value greater than the threshold value TH1 is the measured value MVB (NO in S205), the ECU 170 starts to drive the heater 30B. (S212). Subsequently, the ECU 170 determines whether or not the measured value MVB becomes greater than the threshold value TH2 (S217). When the measured value MVB becomes greater than the threshold value TH2 (YES in S217), the process proceeds to S220. When the measured value MVB does not become greater than the threshold value TH2 (NO in S217), the process proceeds to S230.

As described above, according to this modification, it is possible to reduce the electric power consumption of the heater unit 121.

### Other modifications

The sensor unit 110 is configured to include both sensors 55A, 55B; however, the sensor unit may not include these two sensors but may include a single sensor that measures the concentration of the hydrogen sulfide gas inside the battery case 102. In this case, the ECU 170 starts to drive the heater unit 121 (e.g., the heater 30A) when the measured value of this sensor becomes greater than the threshold value TH1. After starting the driving of the heater unit 121, the ECU 170 determines whether or not this measured value becomes greater than the threshold value TH2. When this measured value does not become greater than the threshold value TH2, the ECU 170 determines that the hydrogen sulfide gas is not generated inside the battery case 102. On the other hand, when the measured value becomes greater than the threshold value TH2, the ECU 170 determines that the hydrogen sulfide gas is generated and executes the responsive control.

It should be considered that the embodiments disclosed herein are illustrative in all respects and are not restrictive. The scope of the present invention is indicated by the claims rather than the above description, and it is intended that all changes within the meaning and the scope equivalent to the claims are included.

## Claims

1. A battery system comprising:
a battery unit accommodated in a battery case, the battery unit including a sulfide-based all-solid-state battery;
a heater unit configured to heat the battery unit; and
a sensor unit configured to measure a hydrogen sulfide gas concentration inside the battery case,
the heater unit being started to be driven when a measured value of the sensor unit becomes greater than a first threshold value,
after starting the driving of the heater unit, when the measured value becomes greater than a second threshold value that is higher than the first threshold value, a responsive control to deal with generation of the hydrogen sulfide gas from the sulfide-based all-solid-state battery being executed.

2. The battery system according to claim 1, wherein
the battery unit includes a first battery and a second battery,
the heater unit includes a first heater that heats the first battery, and a second heater that heats the second battery,
the sensor unit includes a first sensor and a second sensor each of which is configured to measure the hydrogen sulfide gas concentration, the first sensor is disposed in a vicinity of the first battery, and the second sensor is disposed in a vicinity of the second battery, and
when the measured value of the first sensor becomes greater than the first threshold value earlier than the measured value of the second sensor does, only the first heater of the first heater and the second heater is started to be driven.

3. The battery system according to claim 1 or 2, wherein
the battery unit is configured to be chargeable and dischargeable and is configured to be connectable via a relay device to an electric power converter connected to a rotating electric machine that generates propulsive force of an electrified vehicle,
the battery system further includes:
a communication passage that communicates an inside and an outside of the battery case;
an on-off valve provided to the communication passage; and
a notification device that provides notification to a user of the electrified vehicle, and
the responsive control includes at least one of:
opening the relay device;
opening the on-off valve;
controlling the notification device to notify the user of the generation of the hydrogen sulfide gas inside the battery case; and
controlling the electric power converter to restrict charging-discharge of the battery unit.

4. The battery system according to claim 1 or 2, wherein:
the first threshold value is equal to or less than 1 ppm; and
the second threshold value is greater than 1 ppm and less than 5 ppm.
